# EUROPEAN PATENT SPECIFICATION

(11) **EP 3 556 328 B1**
(45) Date of publication and mention of the grant of the patent: **14.02.2024**
(21) Application number: 17881518.9
(22) Date of filing: 31.05.2017
(51) Int. Cl.: A61F 5/02, A61F 5/30

(54) **STERNAL SUPPORT FOR HYPEREXTENSION FRAME**
STERNUMTRÄGER FÜR HYPEREXTENSIONSRAHMEN
APPUI STERNAL POUR CORSET D'HYPEREXTENSION

(30) Priority: 16.12.2016 ES 201631476 U
(43) Date of publication of application: 23.10.2019
(73) Proprietor: Prodigo Inversiones 2010, S.L., 46185 La Pobla de Vallbona (Valencia) (ES)
(72) Inventor: MORTERA DOMENECH, David, 46185 La Pobla de Vallbona (Valencia) (ES)
(74) Representative: Ungria López, Javier
(86) International application number: PCT/ES2017/070375
(87) International publication number: WO 2018/109245

(56) References cited:
- US-A- 2 582 930
- US-A- 3 220 407
- US-A- 3 220 407
- US-A- 3 274 996
- US-A- 5 135 471
- US-A- 6 010 472
- US-B1- 8 574 232

## Description

### Object of the invention

The present invention, as expressed in the title of this specification, refers to a sternal support for a hyperextension frame; wherein said hyperextension frame comprises at least the sternal support, a suprapubic support and a dorsolumbar support which are connected to each other by lateral bars which descend laterally along the midaxillary line. The hyperextension frame has different adjustments in order to be able to adapt correctly to the patient's body; highlighting that the sternal support of the invention enables an adjustment in three spatial directions, substantially improving the adaptation thereof to the body of the patient, thus minimising impacts and allowing adjustment in terms of its elastic amplitude as well as its adaptability to irregular areas of the patient's body.

### Technical problem to be resolved and background of the invention

Hyperextension frames, also called Jewett frames, are currently known which are used to immobilise the spine of a patient, usually after having undergone a surgical operation.

Hyperextension frames generally comprise a first sternal support, a second suprapubic support and a third dorsolumbar support; wherein at least the sternal support has an adjustment system which improves the support on the chest of the patient in order to avoid discomfort and injuries.

However, the known adjustment systems for sternal support do not offer truly effective solutions and also have the drawback of having certain comfort limitations in the sternal support.

The utility model with publication number ES 1057541 U relates to a joint of the hyperextension pelvic band in a jacket which is a Jewett-type or has three support points, which basically describes means for joining a pelvic band (suprapubic support) to each of the two lateral parts which make up the hyperextension jacket (hyperextension frame).

The patent with publication number ES 2353529 T3 relates to a frame for a hyperextension brace comprising a sub-clavicula support and a pelvic support joined by lateral elements: upper and lower, wherein the height of said lateral elements is adjustable by means of a non-rotational telescopic and lockable connection. The sub-clavicula support is connected by adjustable means to the upper lateral elements, and the pelvic and pubic support is connected by adjustable and lockable means to the lower lateral elements; wherein said adjustable and lockable means form a non-rotational telescopic and lockable connection for making adjustments to the width of the hyperextension brace.

The patent with publication number ES 2331668 T3 relates to a frame for a hyperextension clamp in principle comprising a structure similar to the patent mentioned in the previous paragraph, wherein the sub-clavicula support is connected by means of an arrangement of tensioning screws to the lateral elements.

The patent with publication number US3220407A1 may be considered to represent the closest prior art, it describes a surgical brace comprising laterally spaced torso side pads, frame means connecting the side pads and comprising sternal bars and pubic bars connected with the side pads. It also comprises a lumbar pad having strap means connecting it with said frame means, a sternal pad having means connecting it with the sternal bars and a pubic pad having means connecting it with the pubic bars. The sternal bars extends upwardly from the respective side pads and thence extends substantially directly forwardly and curvilinearly toward each other and thence extends upwardly in approximate parallelism to the level of connection with the sternal pad. The sternal pad also comprises an internal plate having an opening and the means connecting it with the sternal bars including a finger extending into said opening, the finger and plate having marginally notched portions in interlocked connection, the plate being self-adjustable with respect to the finger.

### Description of the invention

In order to achieve the objectives and avoid the drawbacks mentioned in the previous sections, the invention proposes a sternal support for a hyperextension frame comprising a mounting combined with a front plate, and damping elements that maintain a separation distance between the mounting and the front plate.

The mounting and the front plate are joined together via attachment devices which include central portions accommodated inside coaxial holes of the damping elements; wherein the front plate can be adjusted in three spatial directions substantially perpendicular to one another.

The front plate comprises slots facing threaded holes located in the mounting; wherein threaded rods of the attachment devices are coupled to the threaded holes of the mounting; wherein portions of the attachment devices fit into the slots of the front plate; and wherein some heads of the attachment devices rest against one of the faces of the front plate.

The front plate includes two slots located in the same longitudinal alignment and the threaded holes of the mounting are located in the same longitudinal alignment.

The damping elements comprise elastic plugs including the through holes wherein the central portions of the attachment devices are accommodated.

In a first embodiment of the invention, the damping elements are in contact with two faces facing each other of the mounting and front plate, while in a second embodiment of the invention washers are also included interspersed between ends of the damping elements and one face of the front plate.

The longitudinal direction wherein the slots are located separates two asymmetric portions of the front plate, such that in this situation said front plate can be located in two different positions with respect to the height of the chest of a patient who is using the hyperextension frame.

Each of the attachment devices comprises a guide body accommodated inside the coaxial hole of the damping element, and a front screw; wherein the guide body includes the threaded rod and a blind perforation in which the previous screw is screwed; and wherein the front screw includes the head of the attachment device and also includes the portion which fits into each slot of the front plate.

The front plate and the mounting are in contact with ends of the guide body of the attachment device; wherein the distance between the front plate and the mounting corresponds to the length of said guide body of the attachment device.

Next, to help better understand this specification and as an integral part thereof, a series of figures is attached in which the object of the invention is depicted in an illustrative and non-limiting manner.

The scope of the invention is defined by the appended claims.

### Brief description of the figures

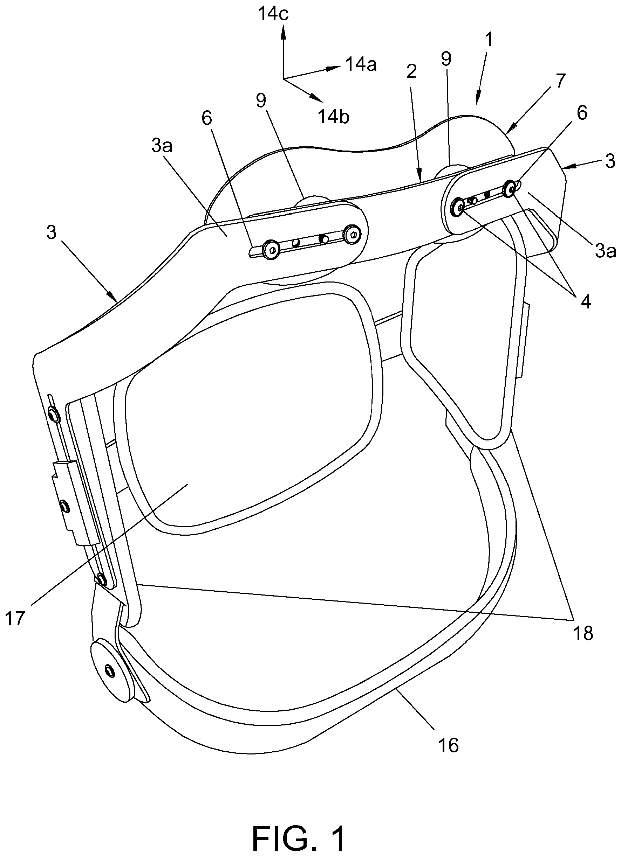

**Figure 1****.-** Shows a perspective view of a hyperextension frame including the sternal support for hyperextension frames, object of the invention.

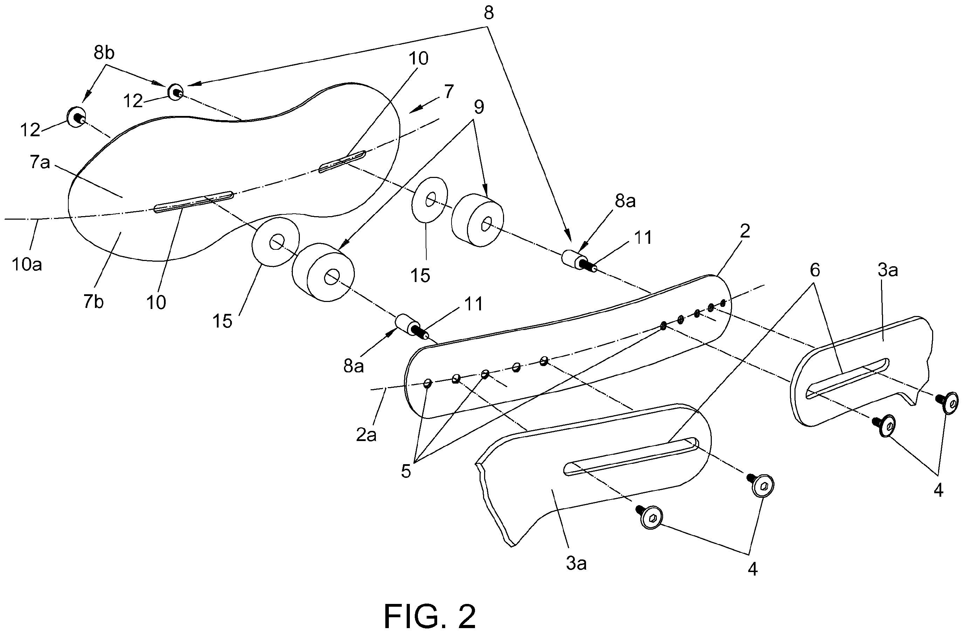

**Figure 2****.-** Shows an exploded view of the sternal support of the invention.
**Figure 3****.-** Shows a cross-sectional view of the sternal support.

### Description of an embodiment of the invention

Pursuant to the numbering adopted in the figures, the sternal support 1 for a hyperextension frame comprises a mounting 2 that is attached to two separate opposed segments 3a making up two lateral reinforcements 3 of the hyperextension frame, wherein said mounting 2 is attached to the two opposed segments 3a by means of rear screws 4 which pass through two elongated recesses 6 located in said opposed segments 3a of the lateral reinforcements 3, and wherein said rear screws 4 are threaded into one of the **several threaded holes 5 located in the mounting 2,** which has a platen structure.

In the embodiment shown in the figures, the threaded holes 5 are located in the same longitudinal alignment 2a of the mounting and distributed in two groups of threaded holes 5 that are facing the elongated recesses 6 located in the two opposed segments 3a of the two lateral reinforcements 3, such that the distribution of the threaded holes 5 in combination with the two elongated recesses 6 enables a certain range of movement in order to position the sternal support 1 assembly in the direction of said elongated recesses 6.

The sternal support 1 of the invention further comprises a front plate 7 which is attached to the mounting 2 by attachment devices 8 including threaded rods 11 which are threaded into others of the various threaded holes 5 of the mounting 2; wherein damping elements 9 are located between the mounting 2 and the front plate 7, such as, for example, elastic plugs made of elastomer material, which make it possible to adjust the relative distance between the mounting 2 and the front plate 7; maintaining at all times a comfortable front damping of the sternal support 1 against the chest of the patient during the use of the hyperextension frame.

In the embodiment shown in the figures, the damping elements 9 are coupled around central portions of the attachment devices 8. For this purpose, said damping elements 9 have coaxial holes 9a wherein the central portions of the attachment devices 8 are adjusted. The damping elements 9 rest on two faces facing each other of the mounting 2 and front plate 7, with interposition of washers 15 located between said front plate 7 and the damping elements 9.

Each of the attachment devices 8 comprises a guide body 8a accommodated inside the coaxial hole 9a of the damping element 9, and a front screw 8b; wherein the guide body 8a includes the threaded rod 11 and a blind perforation 13 wherein the front screw 8b is threaded; and wherein the front screw 8b includes a head 12 resting against one of the faces of the front plate 7.

The front plate 7 comprises two slots 10 arranged in the same longitudinal alignment 10a, through which portions of the front screws 8b making up the attachment devices 8 also pass, such that the heads 12 of said front screws 8b rest against one of the faces of the front plate 7.

The two slots 10 of the front plate 7 enable said front plate 7 to be moved in order to place it in the position required by the patient along a first direction 14a transverse to the width of the chest of the patient, such that once the front plate 7 has been placed in the required position, the attachment devices 8 are then tightened, such that during this tightening operation the positioning of the front plate 7 is also adjusted in a second front direction 14b which is perpendicular to the first direction 14a.

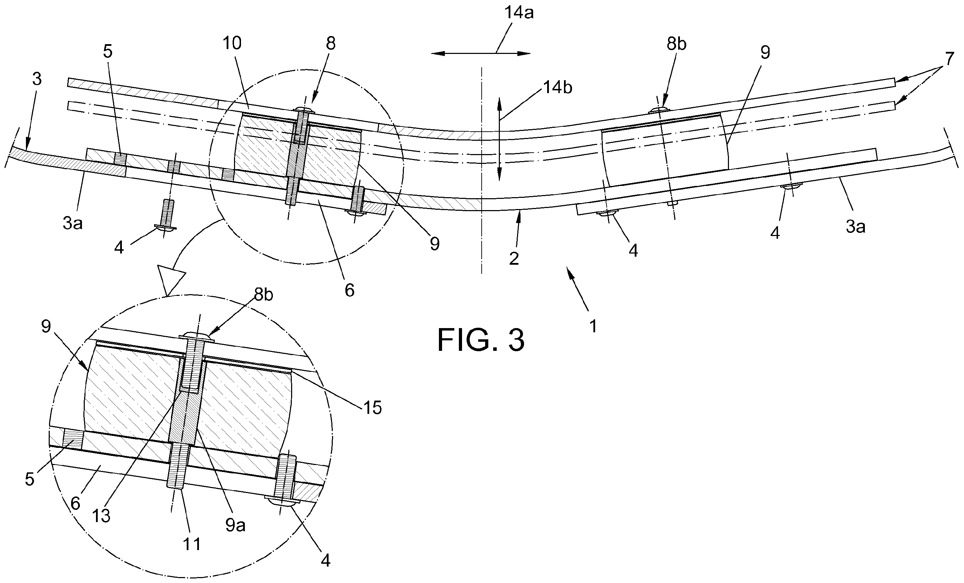

Although the front plate 7 comes closer or moves away depending on the tightening of the attachment devices 8, the final position of said attachment devices 8 is the one shown in figure 3; meaning a final position wherein the front plate 7 and the mounting 2 are in contact with ends of the guide body 8a of the attachment device 8, such that the distance between the front plate 7 and the mounting 2 corresponds to the length of said guide body 8a of the attachment device 8. In this situation, a damping is achieved in the second direction 14b by means of the elastomeric material of the damping elements 9.

The front plate 7 has an adjustment in a third direction 14c which is substantially perpendicular to the other two directions described: first 14a and second 14b, such that by means of this new adjustment in the third direction 14c the relative location of the front plate 7 can be varied, as means for height adjustment.

The adjustment of the front plate 7 in this third direction 14c is achieved by disassembling the front plate 7 and reassembling it in another different position by rotating it 180°, since the longitudinal alignment 10a wherein the two slots 10 are located, separates two asymmetrical portions 7a, 7b of the front plate 7.

The adjustment in this third direction 14c enables the sternal support to be adapted in order to adapt both to a greater height of the patient and to a different anatomy or possible dysmetria of said patient.

Another option for adjusting the front plate 7 in the third direction 14c could consist of including several pairs of slots in the front plate 7 arranged in different longitudinal alignments.

Thus, the sternal support 1 is attached to the two opposed segments 3a of the lateral reinforcements 3 which make up the hyperextension frame, which further includes a suprapubic support 16 and a dorsolumbar support 17. There is also the option of including lateral supports 18 which settle against the sides of the body of the patient.

Moreover, the front plate 7 includes a protective coating not shown in the figures; wherein said protective coating softens the contact of the front plate 7 on the body of the patient.

## Claims

1. **A sternal support for hyperextension frame** that comprises a mounting (2) combined with a front plate (7), wherein the mounting (2) and the front plate (7) are joined together via attachment devices (8),
the sternal support being **characterised in that**:
- it further comprises damping elements (9) with coaxial holes (9a) that maintain a separation distance between the mounting (2) and the front plate (7), and
- the attachment devices (8) include central portions accommodated inside the coaxial holes (9a) of the damping elements (9);
wherein the front plate (7) is adjustable with respect to the mounting (2) in three spatial directions perpendicular to one another.

2. **The sternal support for hyperextension frame,** according to claim 1, **characterised in that**:
- the front plate (7) comprises faces with slots (10),
- the mounting (2) comprises threaded holes (5), and
- the attachment device (8) comprises front screws (8b) with heads (12) and guide bodies (8a) with threaded rods (11) which are configured to be coupled to the threaded holes (5) of the mounting (2);
wherein portions of the attachment devices (8) fit into the slots (10) of the front plate (7); and wherein some heads (12) of the front screws (8b) rest against one of the faces of the front plate (7).

3. **The sternal support for hyperextension frame,** according to claim 2, **characterised in that** the front plate (7) includes two slots (10) located along an alignment (10a) and the threaded holes (5) of the mounting (2) are located along another alignment (2a).

4. **The sternal support for hyperextension frame,** according to any of the preceding claims, **characterised in that** the damping elements (9) comprise elastic plugs which include the through holes (9a) wherein central portions of the attachment devices (8) are accommodated.

5. **The sternal support for hyperextension frame,** according to any of the preceding claims, **characterised in that** the damping elements (9) are in contact with one face of the mounting (2) and another face of the front plate (7), the two faces facing each other.

6. **The sternal support for hyperextension frame,** according to any of claims 1 to 4, **characterised in that** it includes washers (15) interspersed between ends of the damping elements (9) and one face of the front plate (7).

7. **The sternal support for hyperextension frame,** according to claim 3, **characterised in that** the front plate (7) comprises two asymmetrical portions (7a, 7b) that are separated by the longitudinal direction of the alignment (10a) along which the slots (10) are located.

8. **The sternal support for hyperextension frame,** according to claim 2, **characterised in that**:
- the guide body (8a) is accommodated inside the coaxial hole (9a) of the damping element (9) and includes a blind perforation (13) which is threaded, and
- the front screw (8b) includes a threaded portion which fits into the slot (10) of the front plate (7) and is located in the blind perforation (13) of the guide body (8a).

9. **The sternal support for hyperextension frame,** according to claim 8, **characterised in that** the front plate (7) and the mounting (2) are in contact with ends of the guide body (8a) of the attachment device (8); wherein the distance between the front plate (7) and the mounting (2) corresponds to the length of said guide body (8a) of the attachment device (8).

## Patentansprüche

1. Sternumstütze für einen Hyperextensionsrahmen, die einen mit einer vorderen Platte (7) kombinierten Montageteil (2) umfasst, wobei der Montageteil (2) und die vordere Platte (7) durch Befestigungseinrichtungen (8) miteinander verbunden sind,
wobei die Sternumstütze **dadurch gekennzeichnet ist, dass**:
- sie weiterhin Dämpfungselemente (9) mit Koaxiallöchern (9a) umfasst, die einen Trennungsabstand zwischen dem Montageteil (2) und der vorderen Platte (7) aufrechterhalten, und
- die Befestigungseinrichtungen (8) mittlere Teile umfassen, die in den Koaxiallöchern (9a) der Dämpfungselemente (9) aufgenommen sind,
wobei die vordere Platte (7) in Bezug auf den Montageteil (2) in drei zueinander senkrechten räumlichen Richtungen eingestellt werden kann.

2. Sternumstütze für einen Hyperextensionsrahmen nach Anspruch 1, **dadurch gekennzeichnet, dass**:
- die vordere Platte (7) Flächen mit Schlitzen (10) umfasst,
- der Montageteil (2) Gewindelöcher (5) enthält, und
- die Befestigungseinrichtungen (8) vordere Schrauben (8b) mit Köpfen (12) und Führungskörper (8a) mit Gewindestangen (11), die für eine Kopplung mit den Gewindelöchern (5) des Montageteils (2) konfiguriert sind, umfassen,
wobei Teile der Befestigungseinrichtungen (8) in die Schlitze (10) der vorderen Platte (7) passen und wobei einige Köpfe (12) der vorderen Schrauben (8b) gegen eine der Flächen der vorderen Platte (7) ruhen.

3. Sternumstütze für einen Hyperextensionsrahmen nach Anspruch 2, **dadurch gekennzeichnet, dass** die vordere Platte (7) zwei entlang einer Ausrichtung (10a) angeordnete Schlitze (10) enthält und die Gewindelöcher (5) des Montageteils (2) entlang einer anderen Ausrichtung (2a) angeordnet sind.

4. Sternumstütze für einen Hyperextensionsrahmen nach einem der vorstehenden Ansprüche, **dadurch gekennzeichnet, dass** die Dämpfungselemente (9) aus elastischen Zapfen bestehen, die die Durchgangslöcher (9a) enthalten, in denen mittlere Teile der Befestigungseinrichtungen (8) aufgenommen sind.

5. Sternumstütze für einen Hyperextensionsrahmen nach einem der vorstehenden Ansprüche, **dadurch gekennzeichnet, dass** die Dämpfungselemente (9) in Kontakt mit einer Fläche des Montageteils (2) und einer anderen Fläche der vorderen Platte (7) sind, wobei die zwei Flächen einander zugewandt sind.

6. Sternumstütze für einen Hyperextensionsrahmen nach einem der Ansprüche 1 bis 4, **dadurch gekennzeichnet, dass** diese Unterlegscheiben (15) umfasst, die zwischen Enden der Dämpfungselemente (9) und einer Fläche der vorderen Platte (7) angeordnet sind.

7. Sternumstütze für einen Hyperextensionsrahmen nach Anspruch 3, **dadurch gekennzeichnet, dass** die vordere Platte (7) zwei asymmetrische Teile (7a, 7b) umfasst, die durch die Längsrichtung der Ausrichtung (10a), entlang von welcher die Schlitze (10) angeordnet sind, getrennt sind.

8. Sternumstütze für einen Hyperextensionsrahmen nach Anspruch 2 **dadurch gekennzeichnet, dass**:
- die Führungskörper (8a) in den Koaxiallöchern (9a) der Dämpfungselement (9) aufgenommen sind und jeweils ein mit einem Gewinde versehenes Blindloch (13) enthalten, und
- die vorderen Schrauben (8b) Gewindeteile umfassen, die in die Schlitze (10) der vorderen Platte (7) passen und jeweils in den Blindlöchern (13) der Führungskörper (8a) angeordnet sind.

9. Sternumstütze für einen Hyperextensionsrahmen nach Anspruch 8, **dadurch gekennzeichnet, dass** die vordere Platte (7) und der Montageteil (2) in Kontakt mit Enden der Führungskörper (8a) der Befestigungseinrichtungen (8) sind, wobei die Distanz zwischen der vorderen Platte (7) und dem Montageteil (2) der Länge der Führungskörper (8a) der Befestigungseinrichtungen (8) entspricht.

## Revendications

1. Support sternal pour cadre d'hyperextension qui comprend un châssis (2) combiné à une plaque frontale (7), dans lequel le châssis (2) et la plaque frontale (7) sont reliés entre eux par l'intermédiaire de dispositifs de fixation (8), le support sternal étant **caractérisé en ce que**:
- il comprend en outre des éléments d'amortissement (9) avec des trous coaxiaux (9a) qui maintiennent une distance de séparation entre le châssis (2) et la plaque frontale (7), et
- les dispositifs de fixation (8) incluent des parties centrales logées à l'intérieur des trous coaxiaux (9a) des éléments d'amortissement (9);
dans lequel la plaque frontale (7) est réglable par rapport au châssis (2) dans trois directions spatiales perpendiculaires les unes aux autres.

2. Support sternal pour cadre d'hyperextension, selon la revendication 1, **caractérisé en ce que**:
- la plaque frontale (7) comprend des faces avec des fentes (10),
- le châssis (2) comprend des trous taraudés (5), et
- le dispositif de fixation (8) comprend des vis frontales (8b) avec des têtes (12) et des corps de guidage (8a) avec des tiges filetées (11) qui sont configurées pour être couplées aux trous taraudés (5) du châssis (2);
dans lequel des parties des dispositifs de fixation (8) s'insèrent dans les fentes (10) de la plaque frontale (7); et dans lequel certaines têtes (12) des vis frontales (8b) reposent contre l'une des faces de la plaque frontale (7).

3. Support sternal pour cadre d'hyperextension, selon la revendication 2, **caractérisé en ce que** la plaque frontale (7) inclut deux fentes (10) situées le long d'un alignement (10a) et les trous taraudés (5) du châssis (2) sont situés le long d'un autre alignement (2a).

4. Support sternal pour cadre d'hyperextension, selon l'une quelconque des revendications précédentes, **caractérisé en ce que** les éléments d'amortissement (9) comprennent des tampons élastiques qui incluent les trous traversants (9a) dans lesquels sont logées des parties centrales des dispositifs de fixation (8).

5. Support sternal pour cadre d'hyperextension, selon l'une quelconque des revendications précédentes, **caractérisé en ce que** les éléments d'amortissement (9) sont en contact avec une face du châssis (2) et une autre face de la plaque frontale (7), les deux faces se faisant face.

6. Support sternal pour cadre d'hyperextension, selon l'une quelconque des revendications 1 à 4, **caractérisé en ce qu'**il inclut des rondelles (15) intercalées entre des extrémités des éléments d'amortissement (9) et une face de la plaque frontale (7).

7. Support sternal pour cadre d'hyperextension, selon la revendication 3, **caractérisé en ce que** la plaque frontale (7) comprend deux parties asymétriques (7a, 7b) qui sont séparées par la direction longitudinale de l'alignement (10a) le long duquel les fentes (10) sont situées.

8. Support sternal pour cadre d'hyperextension, selon la revendication 2, **caractérisé en ce que**:
- le corps de guidage (8a) est logé à l'intérieur du trou coaxial (9a) de l'élément d'amortissement (9) et inclut une perforation borgne (13) qui est taraudée, et
- la vis frontale (8b) inclut une partie filetée qui s'insère dans la fente (10) de la plaque frontale (7) et est située dans la perforation borgne (13) du corps de guidage (8a).

9. Support sternal pour cadre d'hyperextension, selon la revendication 8, **caractérisé en ce que** la plaque frontale (7) et le châssis (2) sont en contact avec des extrémités du corps de guidage (8a) du dispositif de fixation (8); dans lequel la distance entre la plaque frontale (7) et le châssis (2) correspond à la longueur dudit corps de guidage (8a) du dispositif de fixation (8).
